(19) Europäisches Patentamt

European Patent Office

Office européen des brevets

(11) **EP 3 361 420 A1**

(12) **EUROPEAN PATENT APPLICATION**

(43) Date of publication:
**15.08.2018 Bulletin 2018/33**

(51) Int Cl.:
***G06N 3/00*** *(2006.01)*

(21) Application number: **17155529.5**

(22) Date of filing: **10.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **Charlotte Dorothea Wilhelmina, VINKERS**
**5656 AE Eindhoven (NL)**
• **Arlette, VAN WISSEN**
**5656 AE Eindhoven (NL)**
• **Aart Tijmen, VAN HALTEREN**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **AVATAR RELATIONSHIPS**

(57)     Methods and systems for coaching a user. The system and methods of various calculate at least one relationship determinant value based on a user's behavior. A relationship index value that represents the status of the relationship between a user and a virtual coach is calculated from the determinant value(s). At least one parameter of a coaching plan is then adjusted based on the calculated relationship index value.

**EP 3 361 420 A1**

**Description**

TECHNICAL FIELD

Various embodiments described herein relate to methods and systems directed to embodied conversational agents (ECAs) such as virtual coaches and, more particularly but not exclusively, to methods and systems related to virtual coaches that adaptively coach a user based on the status of the relationship between the user and the virtual coach.

BACKGROUND

Embodied conversational agents (ECAs) are computer agents designed to form socio-emotional relationships with their users. ECAs may also be referred to as "relational agents." ECAs are increasingly being used in the field of healthcare to coach users (e.g., patients) in accordance with treatment regimes or plans.

In order for ECAs to succeed in their role as relational agents, they should build and maintain long-term relationships with their users. Moreover, the ECAs should be able to have natural interactions with their users that are non-repetitive and engaging for the user. These relationships are in contrast to one-off conversations that are merely for instrumental reasons (e.g., to explain a document).

However, existing ECAs are typically designed for single, brief interactions with users. A need exists, therefore, for methods and systems that overcome these disadvantages of existing ECAs.

SUMMARY

This summary is provided to introduce a selection of concepts in a simplified form that are further described below in the Detailed Description section. This summary is not intended to identify or exclude key features or essential features of the claimed subject matter, nor is it intended to be used as an aid in determining the scope of the claimed subject matter.

According to the foregoing, it would be desirable to provide methods and systems that gather data regarding a user's behavior related to the relationship between the user and a virtual coach (i.e., an ECA) and that adjust a coaching plan based on the user's (long term) relationship with the virtual coach.

In one aspect, various embodiments relate to a coaching system. The coaching system includes a communications interface for receiving at least one behavior of a user; a processor; and a memory, the memory configured to store instructions for configuring the processor to: provide a virtual coach that interacts with the user in accordance with a coaching plan defined by at least one parameter; derive at least one relationship determinant value from the at least one measured user behavior; calculate a relationship index value based on the at least one derived relationship determinant value; and adjust at least one parameter of the coaching plan based on the calculated relationship index value.

These features are advantageous because the coaching plan updates based on the interactions with the user and, more particularly, based on the user's behavior during the interaction(s). Accordingly, the features of the present invention consider how the user feels about the virtual coach to provide more effective coaching.

In one embodiment, the processor is further configured to derive at least one second relationship determinant value, calculate a second relationship index value, and adjust the at least one parameter of the coaching plan based on the calculated second relationship index value.

These features are advantageous because the system continuously considers the relationship between the user and the virtual coach over multiple interactions (rather than just a single interaction). The virtual coach is therefore better able to provide more effective coaching.

In one embodiment, the at least one received user behavior is one or more of user facial expression, user posture, user gestures, user dialogue characteristics, use of informal language, user eye gaze, user self-disclosure, user emotions expressed in language, user emotions expressed in text, user comments about the virtual coach, user interest in the virtual coach, and user compliance with the virtual coach.

These features are advantageous because the system is therefore able to consider a variety of user behaviors and gestures to assess the relationship between the user and the virtual coach. Accordingly, the system is able to gather several types of information about the user and therefore provide more effective coaching.

In one embodiment, the at least one derived relationship determinant value is initialized to a predetermined value.

This feature is advantageous because the system initially assesses the relationship between the user and the virtual coach without any preexisting knowledge of the user and how the user feels about the virtual coach.

In one embodiment, the relationship index value is calculated based on at least one of a weighted sum of a plurality of derived relationship determinant values and at least one previously calculated relationship index value.

This feature is advantageous because the system considers a previous relationship index value to more accurately assess the relationship between the user and the virtual coach and how the relationship changes over multiple interactions.

In one embodiment, the processor is configured to adjust the at least one parameter of the coaching plan based on the relationship index value being greater than a predetermined threshold or less than a predetermined threshold.

This feature is advantageous because it provides a simple way to adjust the coaching plan. For example, if the relationship index value is sufficiently high (i.e., above a threshold), the processor may adjust the coaching plan to offer a suggestion or recommendation to a user regarding their health.

In one embodiment, the system further includes a database for storing previously derived relationship determinant values and previously calculated relationship index values, wherein the calculated relationship index value is based on at least one of the previously derived relationship determinant values and at least one previously calculated relationship index value, the at least one previously calculated relationship index value is multiplied by a decaying factor.

These features are advantageous because the system considers previous interactions in determining how to adjust the coaching plan. Moreover, the previous relationship index value(s) are multiplied by a decaying factor so that older relationship index value(s) do not weigh too heavily on the current measurement(s).

In one embodiment, the system further comprises at least one sensor for measuring the at least one behavior of the user.

This feature is advantageous because the system may include sensor devices that measure the user's behavior, rather than having sensors controlled or operating external to the system.

According to another aspect, embodiments relate to a method for coaching a user. The method includes receiving, via a communications interface, at least one behavior of a user; providing, via a processor executing instructions stored on a memory, a virtual coach that interacts with the user in accordance with a coaching plan defined by at least one parameter; deriving, via the processor, at least one relationship determinant value from the at least one measured user behavior; calculating, via the processor, a relationship index value based on the at least one derived relationship determinant value; and adjusting, via the processor, at least one parameter of the coaching plan based on the calculated relationship index value.

These features are advantageous because the coaching plan updates based on the interactions with the user and, more particularly, based on the user's behavior during the interaction(s). Accordingly, the features of the present invention consider how the user feels about the virtual coach to provide more effective coaching.

In one embodiment, the method includes deriving, via the processor, at least a second relationship determinant value, calculating a second relationship index value, and adjusting the at least one parameter of the coaching plan based on the calculated second relationship index value.

These features are advantageous because the method continuously considers the relationship between the user and the virtual coach over multiple interactions (rather than just a single interaction). The virtual coach is therefore better able to provide more effective coaching.

In one embodiment, the at least one received user behavior is one or more of user facial expression, user posture, user gestures, user dialogue characteristics, use of informal language, user eye gaze, user self-disclosure, user emotions expressed in language, user emotions expressed in text, user comments about the virtual coach, user interest in the virtual coach, and user compliance with the virtual coach.

These features are advantageous because the method is therefore able to consider a variety of user behaviors and gestures to assess the relationship between the user and the virtual coach. Accordingly, the system is able to gather several types of information about the user and therefore provide more effective coaching.

In one embodiment, the at least one derived relationship determinant value is initialized to a predetermined value.

This feature is advantageous because the method initially assesses the relationship between the user and the virtual coach without any preexisting knowledge of the user and how the user feels about the virtual coach.

In one embodiment, the at least one relationship index value is calculated based on at least one of a weighted sum of a plurality of derived relationship determinant values and at least one previously calculated relationship index value.

This feature is advantageous because the method considers a previous relationship index value to more accurately assess the relationship between the user and the virtual coach and how the relationship changes over multiple interactions.

In one embodiment, the processor is configured to adjust the at least one parameter of the coaching plan based on the relationship index value being greater than a predetermined threshold or less than a predetermined threshold.

This feature is advantageous because it provides a simple way to adjust the coaching plan. For example, if the relationship index value is sufficiently high (i.e., above a threshold), the processor may adjust the coaching plan to offer a suggestion or recommendation to a user regarding their health.

In one embodiment, the method further includes storing, in a database, previously derived relationship determinant values and previously calculated relationship index values, wherein the calculated relationship index value is based on at least one of the previously derived relationship determinant values and at least one previously calculated relationship index value, wherein the at least one previously calculated relationship index value is multiplied by a decaying factor.

These features are advantageous because the method considers previous interactions in determining how to adjust the coaching plan. Moreover, the previous relationship index value(s) are multiplied by a decaying factor so that older relationship index value(s) do not weigh too heavily on the current measurement(s).

In one embodiment, the method further includes measuring the at least one behavior of the user via at least one sensor.

This feature is advantageous because the method may use sensor devices that measure the user's behavior, rather than having sensors controlled or operated by another party.

According to yet another aspect, embodiments relate to a computer readable medium containing computer-executable instructions for performing a method for coaching a user. The medium includes computer-executable instructions for receiving, via a communications interface, at least one behavior of a user; computer-executable instructions for providing, via a processor executing instructions stored on a memory, a virtual coach interacting with the user in accordance with a coaching plan defined by at least one parameter; computer-executable instructions for deriving, via the processor, at least one relationship determinant value from the at least one measured user behavior; computer-executable instructions for calculating, via the processor, a relationship index value based on the at least one derived relationship determinant value; and computer-executable instructions for adjusting, via the processor, at least one parameter of the coaching plan based on the calculated relationship index value.

These features are advantageous because the coaching plan updates based on the interactions with the user and, more particularly, based on the user's behavior during the interaction(s). Accordingly, the features of the present invention consider how the user feels about the virtual coach to provide more effective coaching.

BRIEF DESCRIPTION OF DRAWINGS

In order to better understand various exemplary embodiments, reference is made to the accompanying drawings, wherein:

FIG. 1 illustrates a system for coaching a user in accordance with one embodiment;

FIG. 2 illustrates the interface of FIG. 1 presenting a virtual coach in accordance with one embodiment;

FIG. 3 illustrates verbal and non-verbal behavior of a user being communicated to the determinant value module of FIG. 1 in accordance with one embodiment;

FIG. 4 illustrates a table showing exemplary user behavioral inputs and their effect on determinants in accordance with one embodiment;

FIG. 5 illustrates a table showing how various behavioral inputs affect the liking determinant in accordance with one embodiment;

FIG. 6 illustrates a table showing how various behavioral inputs affect the trust determinant in accordance with one embodiment;

FIG. 7 illustrates a table showing how various behavioral inputs affect the dominance determinant in accordance with one embodiment;

FIG. 8 illustrates a set of exemplary user behavioral inputs being weighted and mapped to certain determinants in accordance with one embodiment;

FIG. 9 depicts a flowchart of a method for calculating a relationship index value (RIV) in accordance with one embodiment;

FIG. 10 depicts a flowchart of a method for selecting a virtual coach behavior in accordance with one embodiment;

FIG. 11 illustrates a table of exemplary verbal and non-verbal repair behaviors for the virtual coach in accordance with one embodiment;

FIG. 12 depicts a graph of a relationship index value as a function of time in accordance with one embodiment;

FIG. 13 depicts a flowchart of a method for coaching a user in accordance with one embodiment;

FIG. 14 depicts a flowchart of a method for coaching a user in accordance with another embodiment; and

FIG. 15 illustrates an example of a hardware device for implementing the methods described herein in accordance with one embodiment.

DETAILED DESCRIPTION

Various embodiments are described more fully below with reference to the accompanying drawings, which form a part hereof, and which show specific exemplary embodiments. However, the concepts of the present disclosure may be implemented in many different forms and should not be construed as limited to the embodiments set forth herein; rather, these embodiments are provided as part of a thorough and complete disclosure, to fully convey the scope of the concepts, techniques and implementations of the present disclosure to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one example implementation or technique in accordance with the present disclosure. The appearances of the phrase "in one embodiment" in various places in the specification are not necessarily all referring to the same embodiment.

Some portions of the description that follow are presented in terms of symbolic representations of operations on non-transient signals stored within a computer memory. These descriptions and representations are used by those skilled in the data processing arts to most effectively convey the substance of their work to others skilled in the art. Such operations typically require physical manipulations of physical quantities. Usually, though not necessarily, these quantities take the form of electrical, magnetic or optical signals capable of being stored, transferred, combined, compared and otherwise manipulated. It is convenient at times, principally for reasons of common usage, to refer to these signals as bits, values, elements, symbols, characters, terms, numbers, or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices. Portions of the present disclosure include processes and instructions that may be embodied in software, firmware or hardware, and when embodied in software, may be downloaded to reside on and be operated from different platforms used by a variety of operating systems.

The present disclosure also relates to an apparatus for performing the operations herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each may be coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any particular programming language that is sufficient for achieving the techniques and implementations of the present disclosure may be used. A variety of programming languages may be used to implement the present disclosure as discussed herein.

In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

People develop an interpersonal attitude towards others (such as a relationship partner) and an overall attitude towards the relationship over multiple interactions. These attitudes may change as the relationship evolves over time and over multiple interactions. Relationships between humans and computers can be expected to follow a similar course, especially if the computer looks like and acts like a human. Understanding the relationship between a user and the ECA (also referred to as "virtual coach" in the present application) may allow for the development of ECAs that can more successfully influence the human to perform some course of action (e.g., to take medication more often). Knowledge about the status of the relationship between a user and an ECA, such as the user's attitude towards the ECA, is therefore important to selecting appropriate actions to be executed by the ECA.

In view of the disadvantages of existing ECAs discussed above, it would be desirable for an ECA to dynamically adapt to a user to build a strong and long-term relationship with the user. Such a strong, long-term relationship may facilitate trust in the ECA and the ECA's coaching recommendations, as well as more opportunities for the ECA to influence and support the user with their needs.

ECAs in accordance with various embodiments described herein are therefore able to recognize and adapt to a user's social attitude in real-time and during the course of a relationship. More specifically, the ECAs are able to adapt their verbal and non-verbal behavior during interactions with the user to improve engagement with the ECA and improve the relationship over time.

The systems of various embodiments may include one or more processors to process data regarding the user's verbal and non-verbal behavior during interactions with a virtual coach. The processor(s) may then use these values to calculate a relationship index value (RIV). The RIV is a single value summarizing the status of the relationship between the user and the ECA.

Based on the RIV, one or more parameters of a coaching plan may be adjusted. For example, a higher RIV indicates the status of the relationship between the user and the virtual coach is positive, and therefore the user is likely to be receptive and compliant with instructions from the virtual coach. A lower RIV, on the other hand, may indicate the user is distrustful of the virtual coach and may be unlikely to follow instructions from the virtual coach.

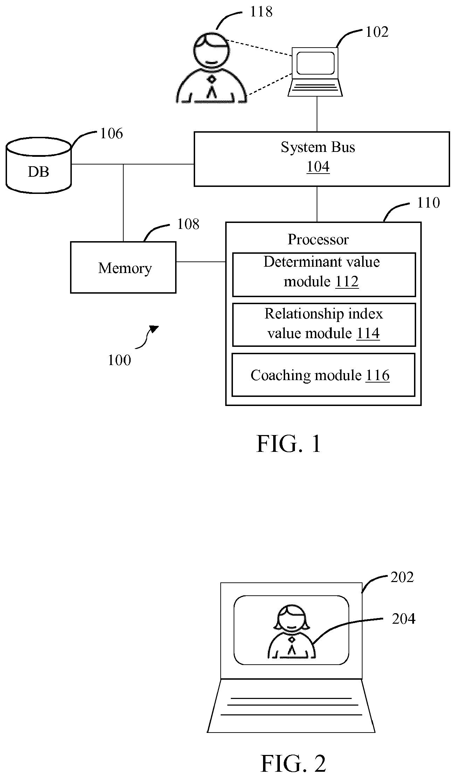

FIG. 1 illustrates an exemplary system 100 for coaching a user in accordance with one embodiment. The system 100 may include an interface 102, a system bus 104, a database 106, memory 108, and a processor 110. The processor 110 may further include a determinant value module 112, a relationship index value (RIV) module 114, and a coaching module 116. The system 100 may be accessible by a user 118 to gather data regarding the user's behavior as well as to coach the user 118.

The interface 102 may be any sort of device that can receive or otherwise gather information regarding a user's behavior and provide coaching instructions to the user. The interface 102 may include a display and may be configured as a PC, laptop, tablet, mobile device, smartwatch, smart TV, or the like. FIG. 2 illustrates an exemplary interface 202. In this embodiment, the interface 202 is configured as a laptop.

The interface 202 may present a virtual coaching avatar 204 (hereinafter "virtual coach"). The virtual coach 204 may communicate instructions to a user in accordance with a coaching plan defined by at least one parameter. As shown in FIG. 2, the virtual coach 204 may be similar in appearance to a human and/or cartoon to make the user feel more comfortable in dealing with the virtual coach 204. For example, when logging into the system 100, the user 118 may be presented with a plurality of options for virtual coaching avatars. These may include avatars of different genders that vary in appearance. The user 118 may select a particular avatar as they wish.

The interface 102 may also include one or more sensors to gather data regarding the behavior of the user 118. For example, the interface 102 may be configured with one or more camera devices to gather pictures and/or videos of the user 118. These types of camera devices may include LIDAR, stereoscopic cameras, eye tracking cameras, infrared cameras, or any other type of camera that can gather information regarding the user, whether available now or invented hereafter. These types of camera devices may gather user behavior information such as whether the user 118 is smiling, frowning, nodding, shaking their head, rolling their eyes, crossing their arms, looking at the virtual coach, looking away from the virtual coach/interface, the temperature of the user's face, or the like. The interface 102 may also include a microphone device to gather auditory-based information from the user 118, such as the contents or tone of the user's speech. The interface 102 may also include heart rate sensor devices to gather information regarding the user's heart rate. It is also contemplated that sensors external to or separate from the interface 102 may measure the behavior of the user, and then communicate data regarding the behavior to the interface 102.

Referring back to FIG. 1, the system bus 104 may enable information to be transferred among the various devices of the system 100. For example, the system bus 104 may communicate data regarding the user's behavior from the interface 102 to the processor 110.

The database 106 may store various types of data in a variety of different formats. This data may include data regarding one or more users such as previously derived relationship determinant values (discussed below), previously calculated relationship index values (discussed below), recorded sensor data, and data regarding a particular user's coaching plan and the user's progress in following said coaching plan.

The memory 108 may be L1, L2, L3 cache or RAM memory configurations. The memory 108 may include non-volatile memory such as flash memory, EPROM, EEPROM, ROM, and PROM, or volatile memory such as static or dynamic RAM, as discussed above. The exact configuration/type of memory 108 may of course vary as long as information such as instructions related to modeling the user's behavior and tailoring at least one parameter of the coaching plan can be stored and executed.

The processor 110 may be any hardware device capable of executing instructions stored on memory 108 to process data regarding the user's behavior. The processor 110 may be a microprocessor, a field programmable gate array (FPGA), an application-specific integrated circuit (ASIC), or a similar type of device. In some embodiments, such as those relying on one or more ASICs, the functionality described as being provided in part via software may instead be configured into the design of the ASICs and, as such, the associated software may be omitted.

As mentioned above, the processor 110 may include a determinant value module 112, a RIV module 114, and a coaching module 116. These components may process the data regarding the user's behavior and tailor and carry out a coaching plan based on the user's behavior.

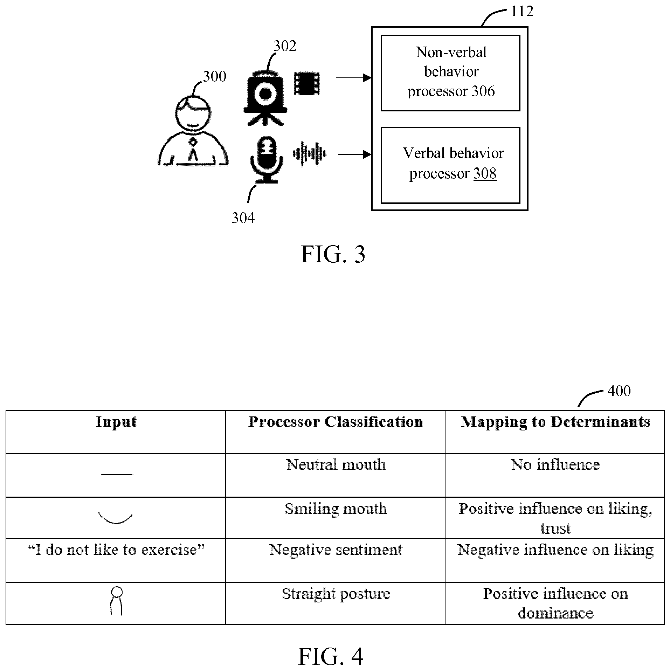

The determinant value module 112 may process the data regarding the user's behavior and derive the determinant values. This may include verbal behavior and/or non-verbal behavior as discussed above. For example, FIG. 3, illustrates a user 300 interacting with a system through a camera device 302 and a microphone device 304. Data regarding the user's behavior captured by the devices 302 and 304 may be communicated to the determinant value module 112. The determinant value module 112 may include a non-verbal behavior processor 306 and a verbal behavior processor 308 to process non-verbal behavior and verbal behavior, respectfully.

Specifically, the determinant value module 112 may use data regarding the user behavior to derive values for at least three relationship determinants. These three exemplary relationship determinants are (1) dominance - the extent to which influence can be exerted on the other; (2) liking - the feeling of attraction or fondness towards the other; and (3) trust - the positive expectation(s) about the dependability of the other. These three determinants, each by themselves or in combination, are known in literature and are considered to be essential in the development of relationships through communication between humans. Additionally, these three relationship determinants play an important role in persuasive communication. In coaching a user, persuasive abilities by the virtual coach is an important criterion to yield beneficial user outcomes because the virtual coach needs to be able to influence the user to meet the user's needs.

The values of each of these determinants are derived from user behavior (such as dialogue between the virtual coach and the user), and may be updated after each interaction or at times during interactions. Behavior related to the user relationship during each conversation may be annotated, recorded, and stored in the database 106. The database 106 may therefore store values for each of these determinants over time and over a plurality of interactions between the user and the virtual coach.

It is noted that these three determinants (dominance, liking, and trust) are one set of minimal, but not exhaustive, building blocks of establishing a relationship between the user and the virtual coach. These three determinants are representative of the relationship, rapport building and its course over time; both between humans and between humans and computers. These determinants are actionable for the system to base decisions on and they can be reliably measured from user verbal behavior and user non-verbal behavior. In other embodiments, other sets of determinants may be used.

The determinant value module 112 may consider any of a plurality of different sensor inputs regarding the user's behavior that contribute to the determinant values. The non-verbal behavior processor 306 may classify non-verbal indicators such as facial expressions, posture, heart rate, gestures, or other types of movements or visual indicia. The verbal behavior processor 308 may similarly classify verbal behavior, such as responses to questions, as either positive or negative. The devices 302 and 304 may continuously and substantially in real time observe the user's behavior and use supervised and/or unsupervised machine learning techniques to learn user behavioral patterns.

The processors 306 and 308 may map each classified input to one or more determinants. For example, FIG. 4 illustrates a table 400 that lists the mapping of various user behavioral inputs, their classifications, and their effect on one or more determinants. An exemplary behavioral input may be that the user is smiling. Using any of computer vision techniques, machine learning, and previously stored data regarding this user, the non-verbal behavior processor 306 may classify this behavioral input as a "smiling mouth." As seen in the table 400, this input behavior and classification has a positive influence on the liking determinant and the trust determinant.

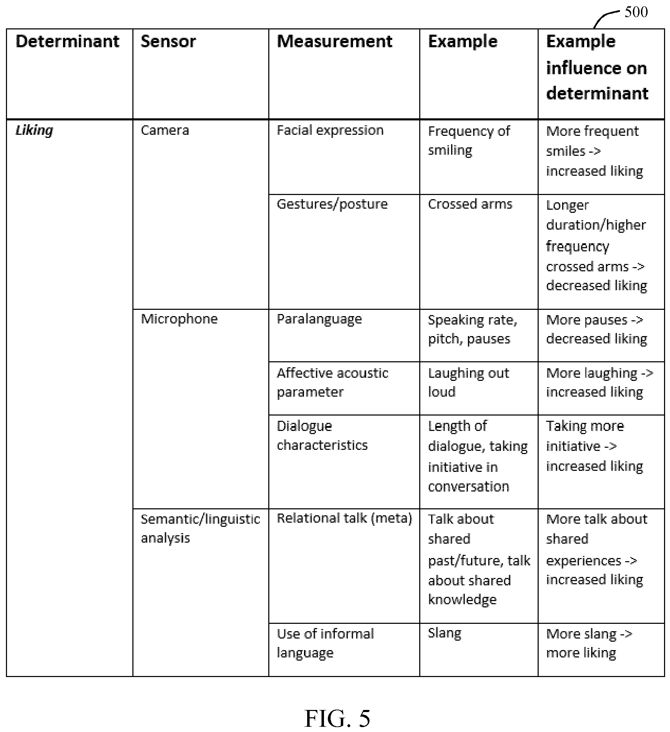

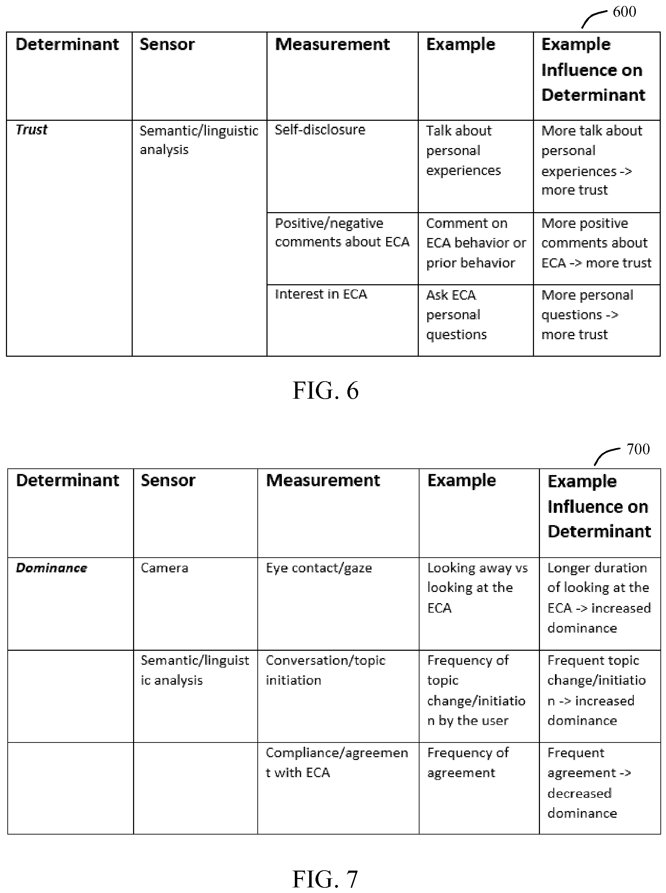

The behavioral inputs of FIG. 4 are merely exemplary, and there may be significantly more types of inputs that may be considered in accordance with the various embodiments described herein. For example, FIG. 5 illustrates a table 500 of examples of behavioral inputs (measurements) and their effect on the liking determinant, FIG. 6 illustrates a table 600 of examples of behavioral inputs and their effect on the trust determinant, and FIG. 7 illustrates a table 700 of examples of behavioral inputs and their effect on the dominance determinant. It is contemplated that other behavioral inputs may be considered as well.

Some behavioral inputs may have more of an effect on or otherwise contribute to determinant values more than other behavioral inputs. The effect an input has on a determinant value is determined by weight values assigned to an input in the RIV calculation. The weighting values assigned to the inputs may be derived from literature and/or from aggregated data from previously learned models for one or more users. Over time, however, machine learning techniques may be employed to learn about interactions and behaviors for each individual user.

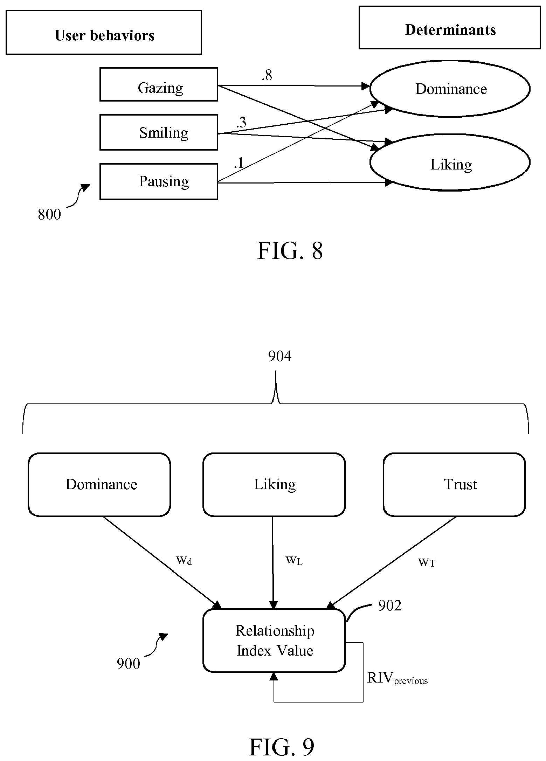

FIG. 8 illustrates an exemplary weighting 800 of user behaviors that are mapped to determinants. In this embodiment, the user behavior inputs gathered are gazing, smiling, and pausing. In this weighting, for example, the input behavior of gazing is assigned a weight of .8 for its effect on both of the dominance and liking determinants. This indicates that the behavior of gazing has more of an effect on the dominance and liking determinants than smiling does (which is assigned a weight of .3). As mentioned above, the weights assigned may initially be developed from literature as well as aggregated data from previously learned models for other users. Over time and over multiple interactions, machine learning techniques may be employed to learn about unique interactions between determinants for each individual.

As can be seen in the tables of FIGS. 5-7 and the weighting 800 of FIG. 8, the majority of input behaviors are often not uniquely linked to one determinant but typically contribute to different determinants. For example, smiling signals both liking and trust, and may affect the determinant values of liking and trust accordingly. Accordingly, the determinants may not independent of one another. As another example, trust may be correlated to dominance in that trust is often considered a prerequisite to display behavior in accordance with a person's dominance predispositions.

The effect of input behaviors on determinants may also depend on the presence and absence of other input behaviors. For example, direct gazing alone can be an indicator of liking. This may increase the liking determinant with weight $w_1$. Direct gazing combined with advancing posture may increase the liking determinant with weight $w_2$ and increase the trust determinant with weight $w_3$.

In operation, the values for each determinant may be initialized to a predetermined value. For example, the dominance determinant $D_d$ may be set to 1, the liking determinant $D_L$ may be set to 1, and the trust determinant $D_t$ may be set to 3.

The determinant values and their respective weights may then be communicated to the relationship index value (RIV) module 114 to calculate the relationship index value (RIV). The RIV is a numeric value (e.g., between 0 and 10) that may be used to select virtual coaching actions that influence the relationship between the virtual coach and the user. In some embodiments the RIV is initially equal to the sum of the products of the determinants and their respective weights (i.e., RIV = $w_d$*Dominance + $w_L$*Liking + wt*Trust). It is noted that the weights should add up to 1. In this embodiment, the RIV for a particular user may be initialized to a predetermined value (which is of course dependent on the predetermined, initialized values of the weights and determinants).

Accordingly, the RIV may be based on the different sources of data (verbal and non-verbal user behavior as indicators of relationship status) and may be calculated and stored each time the user accesses or otherwise interacts with the virtual coach. As the RIV is based on determinants that capture the history of the conversation overtime, the RIV changes in accordance with changes in user behavior over time.

FIG. 9 depicts a flowchart 900 for calculating the RIV in accordance with some embodiments. As can be seen, the relationship index value 902 is dependent on the determinants 904 and their respective weights $w_d$, $w_L$, and $w_T$. After an initial value of the RIV is calculated or set, subsequent RIVs may also be based on previously calculated RIVs. Accordingly, the computation of current RIV scores based, in part, on historic RIV scores helps build and monitor a long term relationship between the user and the virtual coach over multiple interactions.

In operation, an RIV that is dependent only on the determinants and their weights at that particular time may be designated as $RIV_{now}$. That is, $RIV_{now} = w_d$*Dominance + $w_L$*Liking + $w_T$*Trust, wherein $w_d$ is the weight for dominance at that particular time, "Dominance" is the value for the dominance determinant value at that particular time, $w_L$ is the weight for the liking determinant at that particular time, "Liking" is the value for the liking determinant at that particular time, $w_T$ is the weight for the trust determinant at that particular time, and "Trust" is the value for the trust determinant at that particular time. In other words $RIV_{now}$ does not take into account previously calculated determinants or previously calculated RIVs.

After the first RIV calculation, however, the virtual coach takes into account the previously calculated RIVs, as well as $RIV_{now}$. This value may be referred to as the overall RIV, and is calculated as follows:

$$RIV_{Overall} = (1 - Update\_Speed)*RIV_{previous} + Update\_Speed*RIV_{now} \qquad (equation\ 1)$$

The Update Speed may refer to how often the RIV is calculated. The $RIV_{now}$ may be calculated several times during a given interaction between the user and the virtual coach, or only once after the conclusion of an interaction between the user and the virtual coach. As can be deduced from this equation, the larger the update speed the more emphasis is placed on recent interactions. This way of capturing determinant values ensures that the history of the values over past interactions is taken into account. The value "1 - Update Speed" may be considered as a decaying factor that represents how much previous RIVs contribute to the overall RIV. In addition to or instead of considering the Update Speed, various embodiments may multiply previous RIVs with, for example, values less than 1. The higher the decaying factor, the more that previous RIV(s) will contribute to the overall RIV.

Accordingly, the RIV is not only based on current indicators of users' social stances, but is also based on the development and course of the relationship between the user and the virtual coach over time. The $RIV_{Overall}$ and other data may be stored in the database 106 for later retrieval. The $RIV_{Overall}$ may also be communicated to the coaching module 116.

One goal of the system 100 is to adapt the virtual coach's behavior, and therefore the coaching plan and how the coaching plan is conveyed, based on the status of the relationship between the user and the virtual coach. For instance, if the user feels distrustful towards the virtual coach, it may not be wise for the virtual coach to instruct the user to take medication as the user may not comply with the instruction and may even feel resistant towards the request. However, the user may grow to trust the virtual coach over several interactions. This will allow the virtual coach to have more influence over the user. Accordingly, the coaching module 116 may steer the virtual coach's actions before each conversation by selecting the appropriate topics, utterances, gestures, and suggestions that are suitable for the user based on the current relationship status as determined by the $RIV_{Overall}$. Additionally or alternatively, the coaching module 116 may steer the virtual coach's actions during an interaction in a dynamic manner.

The coaching module 116 may be in communication with one or more databases 106 and one or more memories 108 that store instructions or commands for virtual coach behavior. For example, the memories 108 may store instructions for certain virtual coach actions to be executed if the user is feeling distrustful towards the virtual coach.

As another example, the database 106 may store data regarding a particular user, their health, medicinal prescriptions, health plans, or the like. If the user is feeling adequately trustful of the virtual coach, the memory 108 may execute instructions to have the virtual coach instruct the user to take their medicine, for example.

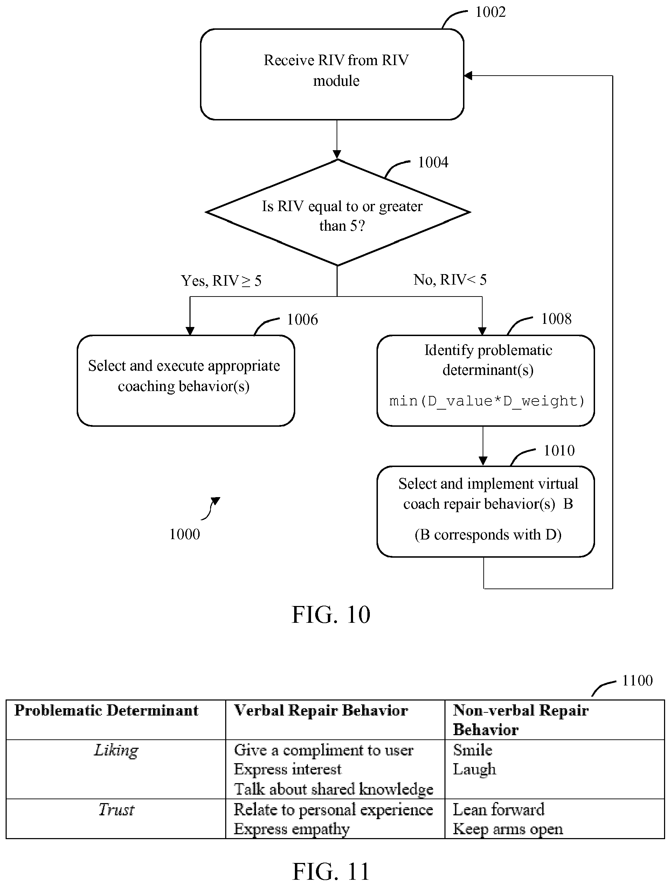

FIG. 10 depicts a flowchart 1000 of a method for selecting a virtual coaching behavior in accordance with one embodiment. Step 1002 involves receiving the RIV value from the RIV module. This may be the current RIV or the overall RIV that considers previously calculated determinant values and RIVs. The RIV may be received at the end of each interaction between a user and the virtual coach or several times throughout an interaction. The particular actions of the virtual coach may depend on the RIV being above or below a threshold level.

Step 1004 therefore considers whether the RIV received in step 1002 is lower or higher than a threshold value of 5. However, the threshold may be set at a value lower or higher than 5. This can also be a generic or personalized threshold. In this particular embodiment, step 1004 considers whether the RIV is greater than or equal to 5. An RIV greater than or equal to 5 may indicate the user has overall positive feelings towards the virtual coach and is therefore likely to be receptive to coaching instructions.

If the overall RIV is greater than or equal to 5, the method 1000 may proceed to step 1006 (in what may be referred to as the coaching phase). Step 1006 involves selecting and executing one or more appropriate coaching behaviors in accordance with a coaching plan.

The selected behavior(s) are predefined as part of the coaching plan and may depend on the RIV. That is, each step of the coaching plan may have preconditions with respect to the RIV as well as with values of individual determinants that need to be satisfied in order for the particular step to be executed by the virtual coach.

In one example, a coaching plan may aim to help a user quit smoking. In order for the virtual coach to persuade the user to quit smoking, the coaching plan may require the overall RIV to be at least 8. Lower overall RIVs, however, may allow the virtual coach to only inform the user about the negative consequences of high blood pressure.

If the RIV is not equal to or above the threshold value of 5 in step 1004, the method 1000 may proceed to step 1008 in what may be referred to as the repairing phase. In this phase, the coaching module 116 may attempt to repair or otherwise improve the relationship because a low RIV may indicate the user may not be receptive towards receiving certain types of instructions, commands, questions, or the like from the virtual coach.

Step 1008 involves identifying one or more problematic determinants. That is, step 1008 considers which of the determinant values are too low and therefore cause the RIV to be lower than the threshold. More complex selection methods that take the weight into account may also be used.

Step 1010 involves selecting and executing one or virtual coach repair behaviors that are based on the problematic determinant. These repair behaviors may include verbal behaviors (what to say and how to say it) and non-verbal behaviors (how to behave) that aim to improve the value of the problematic determinant(s).

FIG. 11 illustrates a table 1100 that specifies verbal and non-verbal behaviors that may be performed by the virtual coach to improve a problematic (low) determinant value. For example, assume the value for the liking determinant is low and therefore prevents the RIV from meeting the threshold. Verbal repair behavior that the virtual coach may take may include one or more of giving a compliment to the user, expressing interest in the user, and talking about shared knowledge. Non-verbal repair behavior that the virtual coach may take may include one or more of smiling and laughing.

After each instance of repair behavior, the method 1000 may proceed back to steps 1002 and 1004 to receive a new RIV and to determine whether the RIV is now equal to or greater than 5. If the overall RIV is equal to or greater than 5 (meaning the repair actions were successful), then the method 1000 may proceed to step 1004. If the overall RIV remains below 5, the method 1000 may proceed to steps 1006 and 1008 and to again attempt to increase the value of the determinant(s). Exemplary pseudocode for this procedure is depicted below:

```
# weights
wd <- 0.3
wl <- 0.3
wt <- 0.4
update_speed <- 0.1

# how dominance develops over time
dominance <-function(t) {
 dominance <- 1
 if (t>250){ dominance <- 10{
 dominance
}

# how liking develops over time
liking <-function(t) {
 liking <- 1 # default
 if (t> 150) liking <- 3}
 if (t>250){ liking <- 10}
 liking
}

trust <- function(t) {
 trust <- 3 # default
 if (t>50) { trust <- 1 }
 if (t>100){ trust <- 3 }
 if (t>120){ trust <- 5}
 if (t>250){ trust <- 10}
 trust
}


riv <- function(riv_old, dom, trust, like){
 (1-update_speed)*riv_old + (wd*dom + wt*trust + wl*like)*update_speed
}

riv_old <- 0
x<- NULL
y<- NULL

for(time in c(0:300)) {
 x <- c(x,time)
 riv_new = riv(riv_old,dominance(time),trust(time),liking(time))
 y <- c(y, riv_new)
 riv_old <- riv_new
}
```

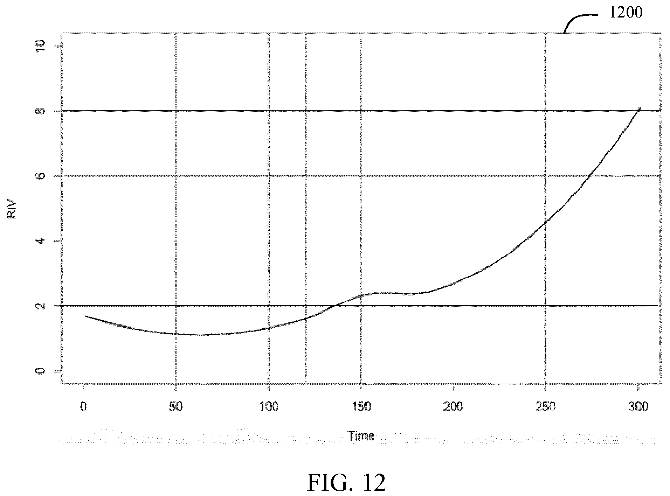

FIG. 12 illustrates a graph 1200 that plots the overall RIV as a function of time that results from the above pseudocode's execution. As can be seen, the overall RIV increases with time. Time may be measured in, for example, seconds, minutes, or days in which the user interacts with the virtual coach and/or the user's behavior is measured.

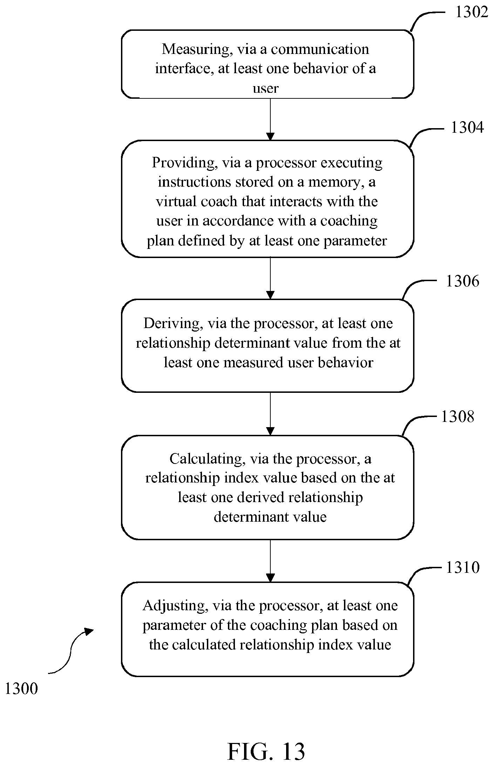

FIG. 13 depicts a flowchart of a method 1300 for coaching a user in accordance with one embodiment. Step 1302 involves measuring, via a communication interface, at least one behavior of a user. The communication interface may be similar to the interface 102 of FIG. 1. The at least one measured behavior may relate to any one of the behavior inputs listed in FIGS. 5-7 (in the "measurement" column), for example, and may include verbal behavior and/or non-verbal behavior. The at least one behavior may be measured by any suitable sensor or input device.

Step 1304 involves providing, via a processor executing instructions stored on a memory, a virtual coach that interacts with the user in accordance with a coaching plan defined by at least one parameter. The processor may be similar to the processor 110 of FIG. 1. The coaching plan may be a generic plan or a plan configured for a specific user. The coaching plan may be aimed towards helping a user improve one or more aspects of their health.

The virtual coach may be presented on an interface such as the interface 102 of FIG. 1, and may be in the form of a person. The at least one parameter may relate to how the virtual coach conveys information (e.g., questions, commands, compliments, instructions, etc.), what information is conveyed to the user, and when such information is conveyed to the user.

Step 1306 involves deriving, via the processor, at least one relationship determinant value from the at least one measured user behavior. The at least one determinant values may relate to the trust, liking, and dominance determinants as discussed previously. Additional determinants and values thereof may also be considered. In some embodiments, for example, an intimacy determinant may be considered as well.

The relationship determinant values may initially be set to a predetermined value. After the virtual coach interacts with the user, however, the user's behavior may have an effect on the determinant value. Generally speaking, the determinant values should increase over time and over multiple interactions with the user.

Step 1308 involves calculating, via the processor, a relationship index value based on the at least one derived relationship determinant value. The relationship index value is a single numeral (e.g., between 0 and 10) that represents the overall state of the relationship between the user and the virtual coach. The higher the RIV, the more positive the user generally feels towards the virtual coach. As seen in FIG. 12, the RIV should increase over time and over multiple interactions.

Step 1310 involves adjusting, via the processor, at least one parameter of the coaching plan based on the calculated relationship index value. The parameter changed may reflect that the RIV is below or above a threshold. For example, the parameter changed may relate to how certain information is conveyed from the virtual coach to the user, what information is conveyed to the user, and when information is conveyed to the user. If the RIV is very low (e.g., a value of 1), the virtual coach may give the user a compliment or engage in small talk rather than issue instructions to the user. In other words, the virtual coach may use information from separate relationship determinants to determine which reparatory actions should be taken to improve the RIV.

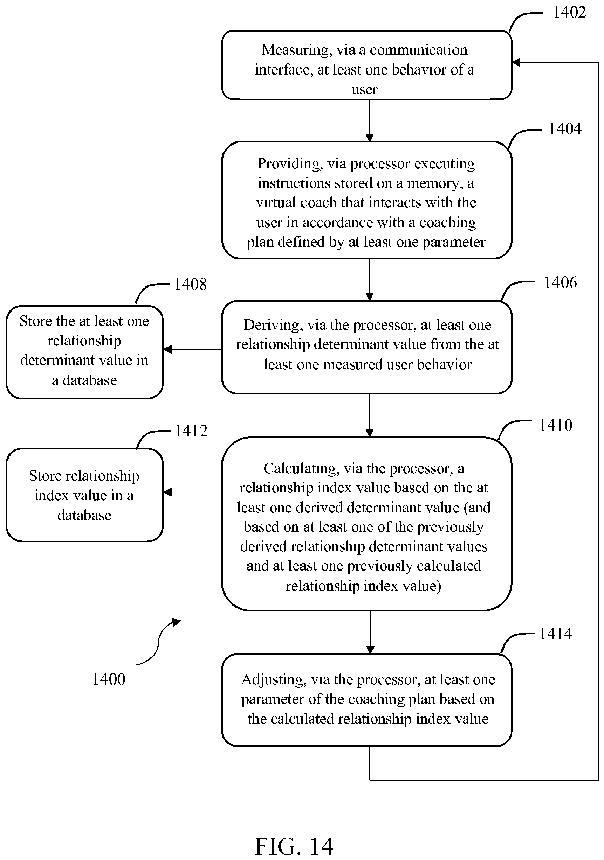

FIG. 14 depicts a flowchart of a method 1400 for coaching a user in another embodiment. Steps 1402, 1404, and 1406 are similar to steps 1302, 1304, and 1206 of FIG. 13, respectively, and are not repeated here. After the at least one relationship determinant value is derived in step 1406, the method 1400 may also proceed to step 1408 which involves storing the at least one relationship determinant value in a database. The database may be similar to the database 106 of FIG. 1, for example. The value may be stored along with a date and time stamp for the particular determinant value.

The method 1400 may then also proceed to step 1408. Step 1408 involves calculating, via the processor, an RIV based on the at least one derived determinant value. Once an RIV is calculated, it may be stored in a database in step 1412 such as the database 106 of FIG. 1.

Step 1414 is similar to step 1310 of FIG. 13 and is not repeated here. Method 1400 may then iterate these steps as many times as desired or required by the user and/or system. However, future iterations may consider previously calculated determinant values and previously calculated RIVs as indicated in step 1410. Accordingly, the method 1400 of FIG. 14 considers previously calculated determinant values and RIVs when calculating future RIVs. The method 1400 therefore considers previous interactions and the relationship between the user and the virtual coach over several interactions rather than just one interaction.

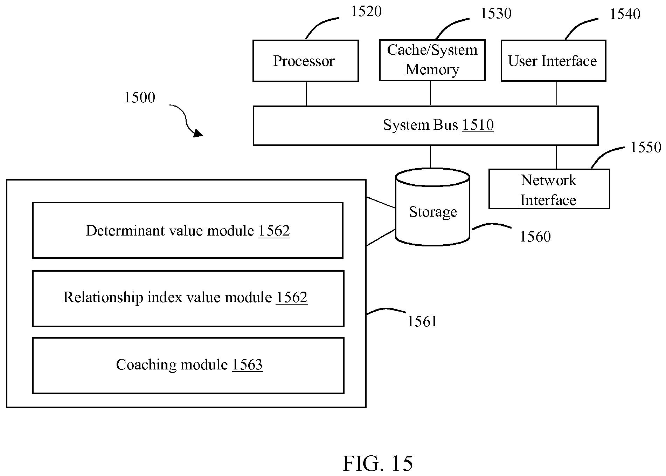

FIG. 15 illustrates an exemplary hardware device 1500 for coaching a user in accordance with one embodiment. As shown, the device 1500 includes a processor 1520, memory 1530, user interface 1540, network interface 1550, and storage 1560 interconnected via one or more system buses 1510. It will be understood that FIG. 15 constitutes, in some respects, a necessary simplification and that the actual organization of the components of the device 1500 may be more complex than illustrated.

The processor 1520 may be any hardware device capable of executing instructions stored in memory 1530 or storage 1560 or otherwise capable of processing data. As such, the processor may include a microprocessor, field programmable gate array (FPGA), application-specific integrated circuit (ASIC), or other similar devices.

The memory 1530 may include various memories such as, for example L1, L2, or L3 cache or system memory. As such, the memory 1530 may include static random access memory (SRAM), dynamic RAM (DRAM), flash memory, read only memory (ROM), or other similar memory devices.

The user interface 1540 may include one or more devices for enabling communication with a user. For example, the user interface 1540 may include a display, a mouse, and a keyboard for receiving user commands and other data pertaining to the user's behavior. In some embodiments, the user interface 1540 may include a command line interface or graphical user interface that may be presented to a remote terminal via the network interface 1550.

The network interface 1550 may include one or more devices for enabling communication with other hardware devices. For example, the network interface 1550 may include a network interface card (NIC) configured to communicate according to the Ethernet protocol. Additionally, the network interface 1550 may implement a TCP/IP stack for communication according to the TCP/IP protocols. Various alternative or additional hardware or configurations for the network interface 1550 will be apparent.

The storage 1560 may include one or more machine-readable storage media such as read-only memory (ROM), random-access memory (RAM), magnetic disk storage media, optical storage media, flash-memory devices, or similar storage media. In various embodiments, the storage 1560 may store instructions for execution by the processor 1520 or data upon with the processor 1520 may operate.

For example the storage 1560 may include an operating system 1561 that includes a determinant value module 1562 for deriving relationship determinant values, a relationship index value module 1562 for calculating a relationship index value based on the derived determinant values, and a coaching module 1563 for executing a coaching plan based on the calculated relationship index value(s).

The methods, systems, and devices discussed above are examples. Various configurations may omit, substitute, or add various procedures or components as appropriate. For instance, in alternative configurations, the methods may be performed in an order different from that described, and that various steps may be added, omitted, or combined. Also, features described with respect to certain configurations may be combined in various other configurations. Different aspects and elements of the configurations may be combined in a similar manner. Also, technology evolves and, thus, many of the elements are examples and do not limit the scope of the disclosure or claims.

Embodiments of the present disclosure, for example, are described above with reference to block diagrams and/or operational illustrations of methods, systems, and computer program products according to embodiments of the present disclosure. The functions/acts noted in the blocks may occur out of the order as shown in any flowchart. For example, two blocks shown in succession may in fact be executed substantially concurrent or the blocks may sometimes be executed in the reverse order, depending upon the functionality/acts involved. Additionally, or alternatively, not all of the blocks shown in any flowchart need to be performed and/or executed. For example, if a given flowchart has five blocks containing functions/acts, it may be the case that only three of the five blocks are performed and/or executed. In this example, any of the three of the five blocks may be performed and/or executed.

A statement that a value exceeds (or is more than) a first threshold value is equivalent to a statement that the value meets or exceeds a second threshold value that is slightly greater than the first threshold value, e.g., the second threshold value being one value higher than the first threshold value in the resolution of a relevant system. A statement that a value is less than (or is within) a first threshold value is equivalent to a statement that the value is less than or equal to a second threshold value that is slightly lower than the first threshold value, e.g., the second threshold value being one value lower than the first threshold value in the resolution of the relevant system.

Specific details are given in the description to provide a thorough understanding of example configurations (including implementations). However, configurations may be practiced without these specific details. For example, well-known circuits, processes, algorithms, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques. Various changes may be made in the function and arrangement of elements without departing from the spirit or scope of the disclosure.

Having described several example configurations, various modifications, alternative constructions, and equivalents may be used without departing from the spirit of the disclosure. For example, the above elements may be components of a larger system, wherein other rules may take precedence over or otherwise modify the application of various implementations or techniques of the present disclosure. Also, a number of steps may be undertaken before, during, or after the above elements are considered.

Having been provided with the description and illustration of the present application, one skilled in the art may envision variations, modifications, and alternate embodiments falling within the general inventive concept discussed in this application that do not depart from the scope of the following claims.

**Claims**

1. A coaching system, the system comprising:

   a communications interface for receiving at least one behavior of a user;
   a processor; and
   a memory, the memory configured to store instructions for configuring the processor to:

provide a virtual coach that interacts with the user in accordance with a coaching plan defined by at least one parameter;

derive at least one relationship determinant value from the at least one measured user behavior;

calculate a relationship index value based on the at least one derived relationship determinant value; and

adjust at least one parameter of the coaching plan based on the calculated relationship index value.

2. The coaching system of claim 1, wherein the processor is further configured to derive at least one second relationship determinant value, calculate a second relationship index value, and adjust the at least one parameter of the coaching plan based on the calculated second relationship index value.

3. The coaching system of claim 1, wherein the at least one received user behavior is one or more of user facial expression, user posture, user gestures, user dialogue characteristics, use of informal language, user eye gaze, user self-disclosure, user emotions expressed in language, user emotions expressed in text, user comments about the virtual coach, user interest in the virtual coach, and user compliance with the virtual coach.

4. The coaching system of claim 1, wherein the at least one derived relationship determinant value is initialized to a predetermined value.

5. The coaching system of claim 1, wherein the relationship index value is calculated based on at least one of a weighted sum of a plurality of derived relationship determinant values and at least one previously calculated relationship index value, wherein the at least one previously calculated relationship index value is multiplied by a decaying factor.

6. The coaching system of claim 1, wherein the processor is configured to adjust the at least one parameter of the coaching plan based on the relationship index value being greater than a predetermined threshold or less than a predetermined threshold.

7. The coaching system of claim 1, further comprising a database for storing previously derived relationship determinant values and previously calculated relationship index values, wherein the calculated relationship index value is based on at least one of the previously derived relationship determinant values and at least one previously calculated relationship index value.

8. The coaching system of claim 1, further comprising at least one sensor for measuring the at least one behavior of the user.

9. A method for coaching a user, the method comprising:

receiving, via a communications interface, at least one behavior of a user;

providing, via a processor executing instructions stored on a memory, a virtual coach that interacts with the user in accordance with a coaching plan defined by at least one parameter;

deriving, via the processor, at least one relationship determinant value from the at least one measured user behavior;

calculating, via the processor, a relationship index value based on the at least one derived relationship determinant value; and

adjusting, via the processor, at least one parameter of the coaching plan based on the calculated relationship index value.

10. The method of claim 9, further comprisingderiving, via the processor, at least a second relationship determinant value, calculating a second relationship index value, and adjusting the at least one parameter of the coaching plan based on the calculated second relationship index value.

11. The method of claim 9, wherein the at least one received user behavior is one or more of user facial expression, user posture, user gestures, user dialogue characteristics, use of informal language, user eye gaze, user self-disclosure, user emotions expressed in language, user emotions expressed in text, user comments about the virtual coach, user interest in the virtual coach, and user compliance with the virtual coach.

12. The method of claim 9, wherein the at least one derived relationship determinant value is initialized to a predetermined value.

**13.** The method of claim 9, wherein the at least one relationship index value is calculated based on at least one of a weighted sum of a plurality of derived relationship determinant values and at least one previously calculated relationship index value.

**14.** The method of claim 9, wherein the processor is configured to adjust the at least one parameter of the coaching plan based on the relationship index value being greater than a predetermined threshold or less than a predetermined threshold.

**15.** The method of claim 9, further comprising storing, in a database, previously derived relationship determinant values and previously calculated relationship index values, wherein the calculated relationship index value is based on at least one of the previously derived relationship determinant values and at least one previously calculated relationship index value, wherein the at least one previously calculated relationship index value is multiplied by a decaying factor.

**16.** The method of claim 9, further comprising measuring the at least one behavior of the user via at least one sensor.

**17.** A computer readable medium containing computer-executable instructions for performing a method for coaching a user, the medium comprising:

computer-executable instructions for receiving, via a communications interface, at least one behavior of a user;
computer-executable instructions for providing, via a processor executing instructions stored on a memory, a virtual coach interacting with the user in accordance with a coaching plan defined by at least one parameter;
computer-executable instructions for deriving, via the processor, at least one relationship determinant value from the at least one measured user behavior;
computer-executable instructions for calculating, via the processor, a relationship index value based on the at least one derived relationship determinant value; and
computer-executable instructions for adjusting, via the processor, at least one parameter of the coaching plan based on the calculated relationship index value.

FIG. 1

FIG. 2

112

302

300

Non-verbal
behavior
processor 306

Verbal behavior
processor 308

304

## FIG. 3

400

| Input | Processor Classification | Mapping to Determinants |
|---|---|---|
| ___ | Neutral mouth | No influence |
| ⌣ | Smiling mouth | Positive influence on liking, trust |
| "I do not like to exercise" | Negative sentiment | Negative influence on liking |
| ⋔ | Straight posture | Positive influence on dominance |

## FIG. 4

500

| Determinant | Sensor | Measurement | Example | Example influence on determinant |
|---|---|---|---|---|
| *Liking* | Camera | Facial expression | Frequency of smiling | More frequent smiles -> increased liking |
| | | Gestures/posture | Crossed arms | Longer duration/higher frequency crossed arms -> decreased liking |
| | Microphone | Paralanguage | Speaking rate, pitch, pauses | More pauses -> decreased liking |
| | | Affective acoustic parameter | Laughing out loud | More laughing -> increased liking |
| | | Dialogue characteristics | Length of dialogue, taking initiative in conversation | Taking more initiative -> increased liking |
| | Semantic/linguistic analysis | Relational talk (meta) | Talk about shared past/future, talk about shared knowledge | More talk about shared experiences -> increased liking |
| | | Use of informal language | Slang | More slang -> more liking |

FIG. 5

600

| Determinant | Sensor | Measurement | Example | Example Influence on Determinant |
|---|---|---|---|---|
| *Trust* | Semantic/linguistic analysis | Self-disclosure | Talk about personal experiences | More talk about personal experiences -> more trust |
| | | Positive/negative comments about ECA | Comment on ECA behavior or prior behavior | More positive comments about ECA -> more trust |
| | | Interest in ECA | Ask ECA personal questions | More personal questions -> more trust |

FIG. 6

700

| Determinant | Sensor | Measurement | Example | Example Influence on Determinant |
|---|---|---|---|---|
| *Dominance* | Camera | Eye contact/gaze | Looking away vs looking at the ECA | Longer duration of looking at the ECA -> increased dominance |
| | Semantic/linguistic analysis | Conversation/topic initiation | Frequency of topic change/initiation by the user | Frequent topic change/initiation -> increased dominance |
| | | Compliance/agreement with ECA | Frequency of agreement | Frequent agreement -> decreased dominance |

FIG. 7

FIG. 8

FIG. 9

1002

Receive RIV from RIV module

1004

Is RIV equal to or greater than 5?

Yes, RIV ≥ 5

No, RIV< 5

1006

Select and execute appropriate coaching behavior(s)

1008

Identify problematic determinant(s)

`min(D_value*D_weight)`

1000

1010

Select and implement virtual coach repair behavior(s)  B

(B corresponds with D)

FIG. 10

1100

| Problematic Determinant | Verbal Repair Behavior | Non-verbal Repair Behavior |
|---|---|---|
| *Liking* | Give a compliment to user<br>Express interest<br>Talk about shared knowledge | Smile<br>Laugh |
| *Trust* | Relate to personal experience<br>Express empathy | Lean forward<br>Keep arms open |

FIG. 11

FIG. 12

1302

Measuring, via a communication
interface, at least one behavior of a
user

1304

Providing, via a processor executing
instructions stored on a memory, a
virtual coach that interacts with the
user in accordance with a coaching
plan defined by at least one parameter

1306

Deriving, via the processor, at least one
relationship determinant value from the at
least one measured user behavior

1308

Calculating, via the processor, a
relationship index value based on the at
least one derived relationship
determinant value

1310

Adjusting, via the processor, at least one
parameter of the coaching plan based on
the calculated relationship index value

1300

FIG. 13

1402

Measuring, via a communication interface, at least one behavior of a user

1404

Providing, via processor executing instructions stored on a memory, a virtual coach that interacts with the user in accordance with a coaching plan defined by at least one parameter

1408

Store the at least one relationship determinant value in a database

1406

Deriving, via the processor, at least one relationship determinant value from the at least one measured user behavior

1412

Store relationship index value in a database

1410

Calculating, via the processor, a relationship index value based on the at least one derived determinant value (and based on at least one of the previously derived relationship determinant values and at least one previously calculated relationship index value)

1400

1414

Adjusting, via the processor, at least one parameter of the coaching plan based on the calculated relationship index value

FIG. 14

FIG. 15

Europäisches
Patentamt

European
Patent Office

Office européen
des brevets

**EUROPEAN SEARCH REPORT**

Application Number

EP 17 15 5529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/193839 A1 (KONINKLIJKE PHILIPS NV [NL]) 8 December 2016 (2016-12-08) * abstract * * paragraph [0015] - paragraph [0032] * * paragraph [0050] * * figures 1,2,5 * ----- | 1-17 | INV. G06N3/00 |

TECHNICAL FIELDS
SEARCHED     (IPC)

G06N

The present search report has been drawn up for all claims

| Place of search | Date of completion of the search | Examiner |
|---|---|---|
| The Hague | 17 July 2017 | Appeltant, Lennert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................
& : member of the same patent family, corresponding
    document

EPO FORM 1503 03.82 (P04C01)

**EP 3 361 420 A1**

## ANNEX TO THE EUROPEAN SEARCH REPORT
## ON EUROPEAN PATENT APPLICATION NO.

EP 17 15 5529

17-07-2017

| Patent document cited in search report | Publication date | Patent family member(s) | Publication date |
|---|---|---|---|
| WO 2016193839 A1 | 08-12-2016 | NONE | |

For more details about this annex : see Official Journal of the European Patent Office, No. 12/82